# EUROPEAN PATENT SPECIFICATION

(11) **EP 2 293 739 B1**
(45) Date of publication and mention of the grant of the patent: **28.12.2016**
(21) Application number: 09750143.1
(22) Date of filing: 22.05.2009
(51) Int. Cl.: A61F 2/24

(54) **HEART VALVE REPAIR DEVICE**
VORRICHTUNG ZUR REPARATUR VON HERZKLAPPEN
DISPOSITIF DE RÉPARATION DE VALVULE CARDIAQUE

(30) Priority: 22.05.2008 GB 0809357
(43) Date of publication of application: 16.03.2011
(73) Proprietor: Punjabi, Prakash, London W12 0HS (GB)
(72) Inventor: Punjabi, Prakash, London W12 0HS (GB)
(74) Representative: Kehoe, Laura Ellen
(86) International application number: PCT/GB2009/050561
(87) International publication number: WO 2009/141665

(56) References cited:
- WO-A-2005/046488
- WO-A-2007/015876
- WO-A-2007/142801
- WO-A2-2006/105084
- US-A1- 2007 208 357

## Description

### FIELD

The present invention relates to a device for repairing and improving the function of incompetent heart valves.

### BACKGROUND

The mitral valve (also known as the bicuspid valve) is one of four heart valves. It is located between the left atrium of the heart, which receives fresh, oxygenated blood from the lungs, and the left ventricle, which pumps the blood out to the body. The mitral valve has two flaps or leaflets, which are shaped somewhat like a parachute and are attached to supporting muscle by tendons (chordae tendineae). The valve opening is surrounded by a fibrous ring known as the mitral valve annulus. A normally functioning mitral valve opens during left atrial contraction, allowing blood to flow into the left ventricle, and closes at the end of atrial contraction to prevent blood from flowing back up into the left atrium during left ventricle systole. However, if a person suffers from mitral valve prolapse, this is not the case.

Mitral valve prolapse is when one or both leaflets of the mitral valve are enlarged and some of the supporting tendons are too long. So, when the heart contracts or pumps, the mitral valve leaflets do not close smoothly or evenly. Instead, part of one or both leaflets collapse backward into the left atrium, which can allow a small amount of blood to leak backward through the valve. This leakage is known as mitral regurgitation.

When the mitral valve is not functioning properly, blood does not flow through the heart or to the rest of the body efficiently. Thus, a mitral valve prolapse condition can leave the patient fatigued and short of breath. It is believed that as many as one in five people over age 55 suffers some degree of mitral valve regurgitation.

Most people with mitral valve prolapse, particularly those without symptoms, do not require treatment. However, patients with severe mitral regurgitation may be advised to undergo surgery since the risk associated with severe mitral regurgitation includes enlargement of the left ventricle (and dilation/deformation of the annulus), which can eventually weaken the heart and prevent it from effectively pumping blood. If regurgitation is left untreated for too long the heart may become too weak for surgery.

At present, the two main surgical options are repair or replacement of the mitral valve. In valve replacement surgery, the damaged mitral valve is replaced by a prosthetic valve, which may be a mechanical or tissue valve. Mechanical valves are typically made of metal and are long-lasting. However, a person with a mechanical valve will need to use an anticoagulant medication, such as Warfarin, for the rest of their life to prevent potentially fatal blood clots from forming on and around the valve. Tissue valves (bioprostheses) are made from animal tissue such as a porcine heart valve. They are less durable than mechanical valves and may need replacement. However, an advantage of the tissue valve is that long-term use of anticoagulant medication is not necessary.

Repair of the mitral valve is always preferable to replacement, since there are fewer side effects for the patient. Moreover, recent reports have suggested that repaired valves last at least as long as replaced valves and may last even longer. A skilled surgeon can surgically modify the original valve to eliminate retrograde blood flow by reconnecting valve leaflets using various repair techniques including: replacing broken or elongated chordae; removing excess valve tissue so that the leaflets can close more tightly; or by using any other known repair technique. These modifications can be supplemented by a complementary repair procedure known as an annuloplasty, in which the surgeon tightens/narrows the mitral valve annulus. In some cases, the only type of repair procedure that is needed is an annuloplasty.

In a typical annuloplasty a prosthetic ring known as an annuloplasty ring is sutured to the dilated annulus to reduce the annulus to its normal size, or to smaller than its normal size in certain cases, to make the valve competent. The annuloplasty ring is usually made from a rigid, semi-rigid or fully flexible material, and may take the form of a partial or complete ring.

An example of an annuloplasty ring is disclosed in WO 2006105084.

A critical and particularly complex step in the operation for the surgeon is determining the size of the annulus accurately so that the appropriate size of annuloplasty ring is implanted in the patient. Current techniques involve measurement of the anterior leaflet as well as the intercommisural or the intertrigonal distance, which requires experience and knowledge.

If the chosen annuloplasty ring is too big, the repair may be unsuccessful in that it may not be durable and the valve may continue to leak. If the chosen annuloplasty ring is too small, narrowing of the valve can occur (mitral stenosis), which may lead to systolic anterior motion (SAM), pulmonary hypertension, atrial fibrillation and congestive heart failure.

Another step in the annuloplasty procedure which involves a degree of surgical subjectivity is suturing the annuloplasty ring to the annulus. Poor or inaccurately placed sutures may affect the success of the valve repair and sutures can be prone to tearing during normal movement of the valve annulus. Moreover, mitral valve repair typically involves 15-20 sutures, each requiring multiple knots, causing suturing to take up a significant proportion of the operating time, thereby increasing the amount of time that a patient is reliant on heart bypass support and increasing the risks associated with a bypass, such as thrombosis.

Given the difficulty associated with correct sizing of the annulus and attachment of the annuloplasty ring to the annulus, mitral valve repair requires an experienced and highly trained cardiac surgeon. A less experienced surgeon will usually opt for a valve replacement rather than annuloplasty, which is technically less difficult than repair and guarantees a competent valve but which in the longer-term is not so beneficial to the patient and can lead to additional complications such as poor long term durability (in the case of a tissue valve) and the need for appropriate anticoagulation (in the case of a mechanical valve).

There is a need to improve upon current annuloplasty rings and methods of implanting and attachment in order to simplify the annuloplasty procedure and to encourage wider uptake of repair and annuloplasty over valve replacement for successful repair of the mitral valve.

### SUMMARY

The present invention resides in an annuloplasty device for use in heart valve tissue repair, comprising an elongate band having a plurality of anchors spaced at predetermined intervals along the length of the band, each anchor being capable of assuming an unengaged and engaged configuration, thereby facilitating attachment of the device to the periphery of an annulus.

Furthermore, the elongate band is formed from an elastic or deformable material, which enables extension of the elongate band by between 5 and 50%, more preferably between 10 and 40%, most preferably between 20 and 30%.

The anchor may be formed from a metal or a polymeric material, preferably a shape memory alloy. Preferably, the shape memory alloy is a nickel-titanium alloy such as Nitinol. Where the anchor is formed of a shape memory alloy, the anchor can be converted from the unengaged configuration to the engaged configuration by applying thermal energy at a temperature above the transition temperature of the alloy. In contrast, the anchor can be converted from the engaged configuration to the unengaged configuration by a reduction of thermal energy. The anchor can also be converted from the unengaged configuration to the engaged configuration or from the engaged configuration to the unengaged configuration by applying mechanical force. Suitably, the mechanical force is applied to the anchor using an anchor-conversion implement.

The anchor may take various forms, for example it may be in the form of a staple, a helical wire, a hook or a screw.

The annulus may be a valve or sphincter, preferably a heart valve, most preferably the mitral heart valve.

Preferably, the plurality of anchors are evenly spaced along the length of the elongate band when in the unengaged configuration. The elongate band may have a plurality of predetermined cleavage positions along its length, advantageously allowing the band to be reduced in length prior to use and/or in situ so as to facilitate positioning of the device within the heart tissue.

A method of conducting a heart valve annuloplasty repair, including attaching a device according to the invention to heart valve tissue, further comprising the steps of:
aligning a first end of the device with a region of valve tissue to be repaired;
implanting an anchor located at the first end of the device into the valve tissue by forcing the anchor to adopt a configuration that engages with the valve tissue;
sequentially implanting each subsequent anchor along the length of the elongate band into the valve tissue until the valve is fully repaired and a 20-50% reduction in heart valve circumference, compared to before commencement of the procedure, has been achieved; and
optionally cutting the elongate band at a predetermined cleavage position located between the last engaged anchor and the next unengaged anchor so as to remove any unimplanted portion of the device is given as an example.

### BRIEF DESCRIPTION OF THE DRAWINGS

Figure 1 shows an annuloplasty device in a first embodiment of the invention; (a) is a side view of the annuloplasty device as a straight band prior to use showing the anchors in the unengaged configuration; (b) is a perspective view of the annuloplasty device as it may be shaped in use showing the anchors in the engaged configuration.
Figure 2 shows the anchors of the annuloplasty device of the first embodiment of the invention in various different shapes ((a) to (e)), both in the unengaged and engaged configuration.
Figure 3 shows the annuloplasty device of the first embodiment of the invention having graduated markings indicating convenient cleavage positions.
Figure 4 shows an annuloplasty device in a second embodiment of the invention; (a) is a perspective view of the annuloplasty device showing the anchors in the unengaged configuration; (b) is a perspective view of the annuloplasty device showing the anchors in the engaged configuration; (c) is an inverted perspective view of (a); (d) is a detailed view of an anchor in the unengaged configuration.

### DETAILED DESCRIPTION

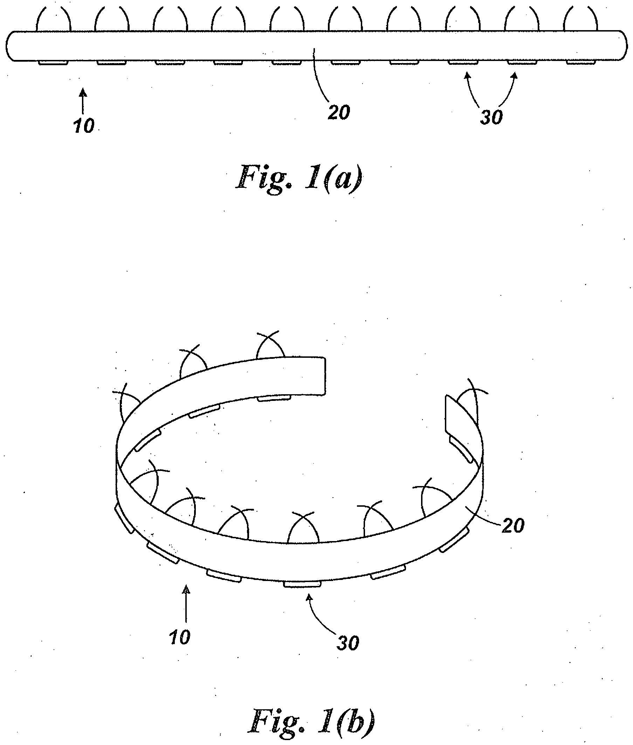

A first specific embodiment of the invention is shown in Figures 1 to 3. As shown in Figure 1, the body of the annuloplasty device 10 is in the form of an elongate band 20 typically made from a flexible elastic, pseudoelastic or deformable material (e.g. a polymer or fabric material) that is extremely long lasting and durable in the body. Suitable materials include woven and non-woven fabrics, elastic cloths such as the polyester fibre Dacron^{®}, polyethylene terephthalate, polyurethane, a thermoplastic elastomer, woven velour, polypropylene, polytetrafluoroethylene (PTFE), heparin-coated fabric, and a silicone or oriented polyethylene. A further alternative embodiment provides for the elongate band 20 to be comprised of non-fabric or polymeric materials such as a superelastic metal or a shape memory alloy such as Nitinol. In yet a further alternative embodiment, the elongate band may be partially or entirely formed from biological tissue, for example, from human or porcine pericardial tissue.

The deformability of the elongate band 20 provides the device 10 with a high degree of overall flexibility which complements the flexibility and variable size and shape of the heart valve annulus during the cardiac cycle and enables the device 10 to match the complex topography of the annulus which can vary from patient to patient. The deformability or elasticity of the elongate band 20 allows the length between each anchor 30 to be extended by between about 5 and 50%, more preferably between 10 and 40%, most preferably 20 and 30%.

The elongate band 20 is provided with a plurality of anchors 30 spaced at intervals along the length of the band 20. The spacing of the anchors 30 can be regular in one embodiment, or in some instances irregular (e.g. increased or reduced density of anchors 30 towards particular regions of the body of the device 10). Typically the distance between adjacent anchors 30 is between about 1 and 10mm, more typically between about 4 and 8mm.

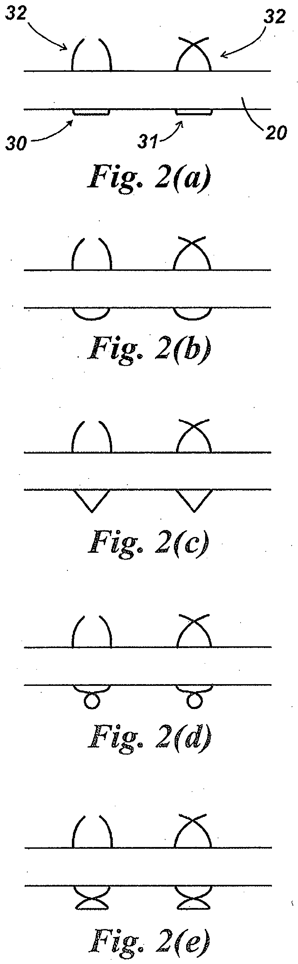

As shown most clearly in Figure 2, the anchor 30 is typically a fastener formed from a looped length of wire and may comprise a base portion 31 which is housed within or on one side of the elongate band 20 and two arms 32 which pass through the body of the elongate band 20 and extend outwardly from the band 20. The arms 32 may initially be straight or curved and typically have sharpened tips to allow the anchor 30 to easily penetrate the annulus tissue and facilitate securing of the device 10 to the annulus.

In use, the anchor 30 can adopt at least two configurations; an unengaged or open configuration (see Figure 1 (a) and the left-hand anchor shown in Figure 2a) to e)) and an engaged or closed configuration (see Figure 1 (b) and the right-hand anchor shown in Figure 2a) to e)). In the unengaged configuration the arms 32 are free and are not attached to the heart valve tissue. In the engaged configuration the arms 32 are implanted into the fibrous tissue of the heart valve annulus, so as to attach to or 'bite' into the tissue and secure the device 10 to the annulus. Typically, the width of the anchor 30 in the unengaged configuration is between 3 and 6 mm, whereas in the engaged configuration it is between 2 and 4 mm. The anchors 30 may be evenly spaced along the length of the elongate band 20 prior to use of the device 10 and when in the unengaged configuration, separated by an intervening region of the elongate band 20.

The number of anchors 30 situated along the length of the elongate band 20 depends upon the initial length of the device 10. The device 10 can be made in a range of different sizes (measured in French units). Typically, a 28F device will contain about twelve anchors.

Suitably, the anchor 30 comprises a shape memory alloy such as a nickel-titanium alloy (e.g. Nitinol) or Elgiloy. In such an instance, the anchor 30 is originally formed in the desired engaged configuration and subsequently cooled below a transition temperature and then formed into the desired unengaged configuration. When in use the device 10 is inserted into the heart valve and the anchor 30 is deployed by the localised application of heat (i.e. thermal energy) at a temperature above the transition temperature. An anchor-engaging implement, a thermal applicator (not shown), is suitably utilised for this purpose. The application of thermal energy can be made to the base portion 31 of the anchor 30 housed within/adjacent to the elongate band 20 thereby insulating the surrounding heart tissue from excessive exposure to heat. Energy can be applied to the anchor 30, for example, via a laser, via a radiofrequency AC electrode, via an ultrasound or microwave emitter, or via an inductive coupling.

In alternative embodiments of the invention, the anchor 30 may be fabricated from other biocompatible metals such as steel, platinum, titanium and gold, or from non-metallic polymeric materials. Where the anchor 30 is formed from a metal or polymeric material other than a shape-memory alloy, it will typically assume the engaged configuration via the application of mechanical force. In a specific embodiment of the invention, an anchor-engaging implement, suitably a surgical stapling tool (not shown) or any other tool, which can contact and apply a mechanical force to the anchor 30, such as via a crimping action, is used for this purpose.

Where the anchor 30 comprises a shape memory alloy, it can be forced to adopt the unengaged configuration by cooling to a temperature below the transition temperature. This may be desirable where the anchor 30 has been implanted at the wrong position and the surgeon wishes to re-implant at another position. The anchor-engaging implement can be used to detach the anchor 30 from the heart tissue by cooling the anchor 30 or by mechanical disengagement. Where the anchor 30 is formed from a metal or polymeric material other than a shape-memory alloy, mechanical disengagement can be used. The ability of the anchor 30 to be manipulated between the unengaged and engaged configurations on multiple occasions can be useful for the operating surgeon during the annuloplasty procedure as it allows for the device 10 to be repositioned if initially implanted incorrectly or at a less desirable position.

In alternative embodiments, fasteners other than the staple-type anchor 30 shown in Figures 1 to 4, can be used. For example, the anchor 30 may incorporate a helical screw, a helical wire, a hook, a serrated barb, a clip, a prong, a butterfly arrangement, a suture pull, a suture tie or may involve a spring mechanism.

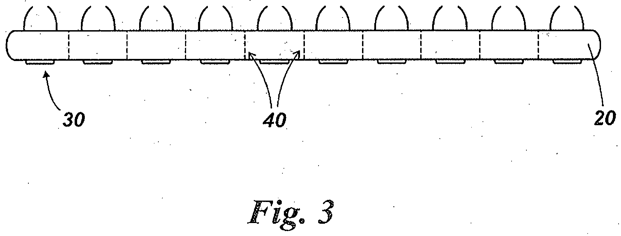

The length of the device 10 of the invention can be reduced prior to use and/or in situ, if necessary, by cleaving at a selected position. As shown in Figure 3, the device 10 may optionally have markings 40 along the length of the band 20 to indicate convenient cleavage positions. This allows the operating cardiac surgeon to tailor the length of the device 10 to the size of the patient's annulus and facilitates positioning of the device 10 in situ. It is a significant advantage of the present invention that a single device 10 can be utilised in virtually all patients and cut to size as needed as, at present, conventional annuloplasty rings are pre-sized. Suitable cutters (not shown) can be used for cleavage of the device 10.

In use, the annuloplasty device 10, as shown in Figures 1 to 3, is inserted into the patient and attached to the annulus in order to repair the heart valve in the following manner. Firstly, one end of the device 10 is aligned with the annulus at an initial anchoring point. The attachment process commences by implanting a first anchor 30 located at a first end of the device 10 into the valve tissue by inducing the anchor 30 to adopt the engaged configuration, either by using thermal energy or mechanical force as appropriate to the design of the anchor 30. Each subsequent anchor 30 along the length of the elongate band 20 can then be sequentially implanted into the annulus until the annulus is reduced in diameter to a point at which the valve is fully repaired.

In order to achieve the desired overall 0-75%, preferably 10-50%, reduction in the circumference of the annulus to effect valve repair, the section of elongate band 20 immediately following the most recently inserted anchor 30, and prior to the next anchor 30 to be inserted, can be extended before the next anchor 30 is implanted. As the section of elongate band 20 retracts under the inherent tensile force of the elastic material from which the device 10 is comprised the adjacent implanted anchors 30 are thereby drawn together and the overall circumference of the valve annulus may be reduced. Whether or not a section of elongate band 20 between adjacent anchors 30 is extended and by how much it is extended is at the discretion of the user and will depend upon the desired extent of annulus circumferential reduction necessary to effect annuloplasty.

In an alternative example, implantation of the device 10 is intended solely to stabilise the periphery of the valve. In this embodiment an effective reduction in valve circumference is not required and, as such, the elastomeric properties of the body of the device 10 (the elongate band 20) are not utilised and the anchors 30 are engaged with the tissue without extension of the portion of the elongate band 20 intervening each anchor 30.

The anchors 30 of the device 10 are typically evenly spaced along the length of the device 10 in a daisy-chain-like manner. However, once the device 10 has been implanted and where some of the sections of elongate band 20 between adjacent anchors 30 have been extended during the process of implantation to effect circumferential reduction of an annulus, the anchors 30 may be unevenly spaced in situ if the elongate band 20 comprises a deformable (e.g. plastic) material as opposed to an elastic material.

The device 10 may be attached to approximately 25-100% of the annulus in order to achieve an effective annuloplastic repair: If attachment of the device 10 to less than 100% of the annulus circumference does not achieve a competently functioning valve, there remains the option of continuing to attach the device 10 around the remaining circumference of the annulus until a competency is achieved. Competency of the valve may be assessed by use of saline solution to determine if the valve reflex is functioning properly.

In the event that a competent valve is achieved without having to implant the entire length of the device 10, the elongate band 20 can be cut between the last engaged anchor 30 and the next unengaged anchor 30 so as to remove any unused part of the device 10. It is possible for the elongate band 20 to be cut at any position between adjacent anchors 30; the presence of these multiple cutting positions allows the surgeon to tailor the length of the device 10 exactly to the size of the individual patient's annulus. In an embodiment of the invention the device 10 comprises predetermined markers 40 (see Figure 3) at positions along the longitudinal axis of the device 10 that indicate where the device 10 can be conveniently cut.

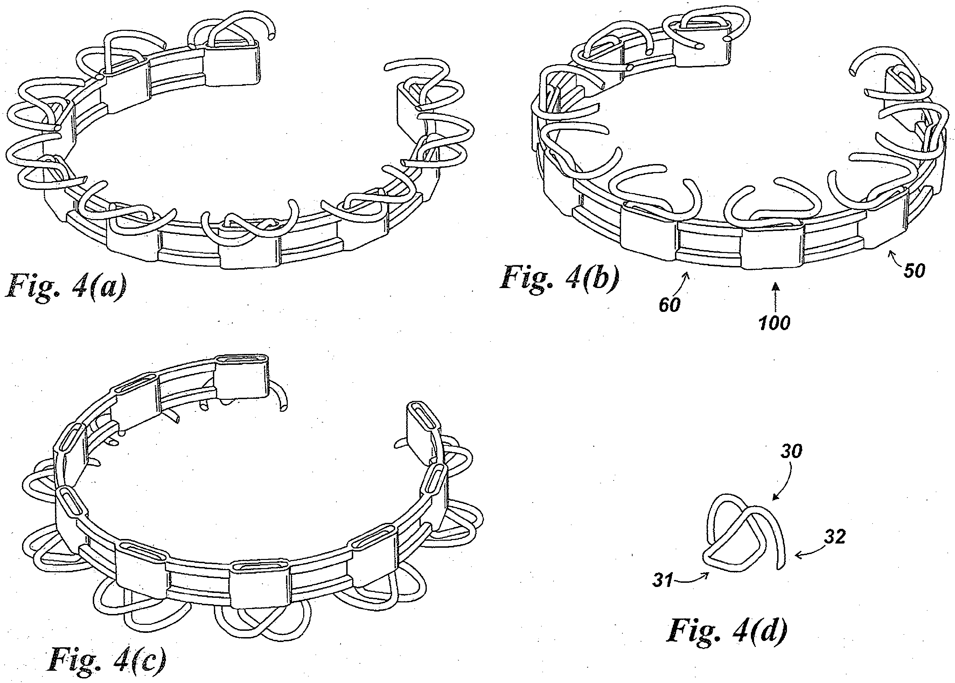

A further specific embodiment of the invention is shown in Figure 4. The annuloplasty device 100 comprises an elongate band comprising a plurality of nodes 50 which are typically made from a flexible elastic or pseudoelastic material that is extremely long lasting and durable in the body, including woven and non-woven fabrics, elastic cloths such as the polyester fibre Dacron^{®}, or from a more inflexible material such as metal or pyrolytic carbon which is thromboresistant. The nodes 50 are connected to each other via linkers 60, which are typically made from an elastic or deformable material, such as polyethylene terephthalate, a linear polyurethane, a thermoplastic elastomer, a silicone or oriented polyethylene, a superelastic metal or a shape memory alloy such as Nitinol, or an elastic woven fabric such as Dacron^{®}. In an alternative embodiment, the linkers and/or nodes may be partially or entirely formed from biological tissue, for example, from human or porcine pericardial tissue.

The elasticity of the linkers 60 allows the distance between each node 50 to be extended by approximately between 1 and 80%, preferably between about 5 and 50%, about 10 and 40%, or about 20 and 30%. This elasticity also provides the device 100 with a high degree of overall flexibility as it enables the linkers 60 to twist and torque relative to the nodes 50. As per the first embodiment of the invention, the flexibility and elasticity of the device 100 complements the flexibility and variable size and shape of the heart valve annulus during the cardiac cycle.

Each node 50 houses an anchor 30, which is shown in more detail in Figure 4(d). The anchor 30 may also take any of the forms shown in Figures 1 to 3. The anchor 30 is formed from a looped length of wire and comprises a base 31 which is housed within or adjacent to the node 50 and two arms 32 which extend outwardly from the node 50. In all embodiments, the arms 32 may extend outwardly in a direction that is either perpendicular to (as shown in Figure 4) or in the plane of (as shown in Figures 1 to 3) the band 20 or node 50. It may also be possible to manually alter the angle of the arms 32 if desired. As per the first embodiment of the invention, the anchor 30 can adopt two configurations, an unengaged configuration (see Figure 4 (a), (c) and (d)) and an engaged configuration (see Figure 4 (b)), and attaches to the heart valve annulus in a corresponding manner. Again, typically, the width of the anchor 30 in the unengaged configuration is between 3 and 6 mm, whereas in the engaged configuration it is between 2 and 4 mm.

The spacing of the nodes 50 and anchors 30 can be regular in one embodiment, or in some instances irregular (e.g. increased or reduced density of anchors 30 towards particular regions of the body of the device 100). Typically the distance between adjacent anchors 30 is between about 1 and 10mm, more typically between about 4 and 8mm.

The materials and shapes of altering the configurations of the anchor are as described in the first embodiment of the invention.

Also, as per the first embodiment of the invention, the length of the elongate band can be reduced prior to use and/or in situ, if necessary, by cleaving at a selected linker position using suitable cutters.

In use, the annuloplasty device 100, as shown in Figure 4, is inserted into the patient and attached to the annulus in order to repair the heart valve in an identical manner to that described in the first embodiment of the invention, except in this instance, in order to achieve the desired overall 0-75%, preferably 10-50%, reduction in the circumference of the annulus to effect valve repair, it is the elastic linker 60 immediately following the most recently inserted anchor 30 that can be extended before the next anchor 30 is implanted. As the linker 60 retracts under the inherent tensile force of the elastic material the adjacent anchors 30 are drawn together and the circumference of the valve annulus is reduced.

Prior to implantation, the nodes 50 and anchors 30 of the device 100 are typically evenly spaced along the length of the elongate band. However, once the device 100 has been implanted and where some or all of the linkers 60 have been stretched during the process of implantation to effect circumferential reduction of an annulus, the nodes 50 and anchors 30 may be unevenly spaced in situ if one or more linkers 60 comprise a deformable (e.g. plastic) material as opposed to an elastic material.

If a competent valve has been achieved without having to implant the entire length of the device 100, the device 100 can be cut at a linker 60 located between the last engaged anchor 30 and the next unengaged anchor 30 so as to remove any unused part of the device 100.

An anchor-engaging implement (not shown) can be used to apply mechanical force to the anchors 30 of the first and second embodiments of the invention to adopt the engaged configuration for implantation into the annulus tissue. It may also be used to effect release of the anchors 30 back into the unengaged configuration in the event that the surgeon wishes to remove the anchor 30 from the annulus tissue and re-implant it at a different position. The anchor-engaging implement may be a suitable heat applicator, cooling applicator or a suitable surgical stapling tool depending on whether the anchor 30 comprises a shape memory alloy or not.

A cutting implement (not shown) can be provided to be used to cut the device of the first and second embodiments of the invention at a selected position and allows the cardiac surgeon to tailor the length of the device to the size of the patient's annulus.

Although the annuloplasty device of the invention is intended for mitral valve repair it would be suitable for repairing any of the four heart valves (mitral, tricuspid, aortic and pulmonary), as well as other heart structures such as a patent foramen ovale, or even structures outside the heart such as the gastroesophageal junction. Hence, in a further embodiment of the invention the annuloplasty device of the invention is directed for use on valve or sphincter repair outside of the heart.

As mentioned previously the annuloplasty device of the invention has the advantage of being able to repair any size of annulus (i.e. one size fits all) and overcomes the problems associated with sizing of the annulus. A typical annuloplasty operation has a preliminary sizing step which requires the use of a purpose made sizing device to determine the size of the annulus so that the correct size of annuloplasty ring is inserted. If the incorrect size of ring is chosen the repair will at best be ineffective and at worst could have serious consequences for the patient. The elongate band design of the device of the invention with predetermined cleavage points along its length, means that this device can be tailored to fit any size of annulus and avoids the need for a time-consuming and difficult sizing step. The fact that the device is initially provided as a substantially straight flexible elongate band, allows the operator to precisely track and adjust the circumference of the annulus during implantation and to resize the device easily and accurately.

The device can be manufactured in a range of different sizes (typically measured in French units). This allows the user to preselect the most suitable sized device for the individual patient, and then to further adjust the length of the device prior to use and/or in situ as appropriate.

A further advantage of the device of the invention is that its sutureless design makes it much simpler and quicker to attach to the annulus of the heart valve, thus substantially reducing operating time.

Although particular embodiments of the invention have been disclosed herein in detail, this has been done by way of example and for the purposes of illustration only. The aforementioned embodiments are not intended to be limiting with respect to the scope of the appended claims. It is contemplated by the inventor that various substitutions, alterations, and modifications may be made to the invention without departing from the scope of the invention as defined by the claims.

## Claims

1. An annuloplasty device (10; 100) for use in heart valve tissue repair, comprising:
an elongate band (20) comprising a flexible elastic or deformable material and having a plurality of anchors (30) spaced at predetermined intervals along the length of the band (20)
wherein the flexible elastic or deformable material of the elongate band enables the length between each anchor (30) to be extended and retracted by between 5 and 50%, **characterised in that** each anchor (30) being capable of assuming an unengaged and engaged configuration, thereby facilitating attachment of the device (10; 100) to the periphery of an annulus.

2. The device according to claim 1, wherein the flexible elastic or deformable material of the elongate band enables the length between each anchor (30) to be extended and retracted by between 10 and 40%, preferably between 20 and 30%.

3. The device according to claim 1 or claim 2, wherein the elongate band (20) is made from a material selected from: woven and non-woven fabrics; elastic cloths, such as Dacron; polyethylene terephthalate; polyurethane; a thermoplastic elastomer; woven velour; polypropylene; polytetrafluoroethylene; heparin-coated fabric; a silicone or oriented polyethylene; or biological tissue.

4. The device according to any previous claim, wherein the anchor (30) comprises a metal or a polymeric material.

5. The device according to any previous claim, wherein the anchor (30) comprises a shape memory alloy.

6. The device according to claim 5, wherein the shape memory alloy comprises Nitinol.

7. The device according to claim 5 or claim 6, wherein the anchor (30) is capable of transition from the unengaged configuration to the engaged configuration by application of thermal energy.

8. The device according to claim 5 or claim 6, wherein the anchor (30) is capable of transition from the engaged configuration to the unengaged configuration by a reduction of thermal energy.

9. The device according to claim 4, wherein the anchor (30) is converted from the unengaged configuration to the engaged configuration or from the engaged configuration to the unengaged configuration by application of mechanical force.

10. The device according to claim 9, wherein mechanical force is applied to the anchor (30) using an anchor-conversion implement.

11. The device according to any previous claim, wherein the anchor (30) comprises a staple.

12. The device according to claim 9 or claim 10, wherein the anchor comprises a helical wire or screw.

13. The device according to any previous claim, wherein the annulus is a valve or sphincter, preferably wherein the valve is a heart valve.

14. The device according to any previous claim, wherein the plurality of anchors (30) are evenly spaced along the length of the elongate band (20) when in the unengaged configuration.

15. The device according to any previous claim, wherein the elongate band (20) comprises a plurality of predetermined cleavage positions (40) along its length, allowing the band (20) to be reduced in length prior to use and/or in situ so as to facilitate positioning of the device (10; 100) within the heart tissue.

## Patentansprüche

1. Anuloplastievorrichtung (10; 100) zum Gebrauch beim Reparieren eines Herzklappengewebes, Folgendes umfassend:
ein längliches Band (20), das ein flexibles elastisches oder verformbares Material umfasst und mehrere Verankerungen (30), die in vorgegebenen Abständen entlang der Länge des Bandes (20) beabstandet sind, aufweist,
wobei das flexible elastische oder verformbare Material des länglichen Bandes es ermöglicht, die Länge zwischen jeder Verankerung (30) um zwischen 5 und 50 % zu verlängern und einzuziehen, **dadurch gekennzeichnet, dass** jede Verankerung (30) in der Lage ist, eine eingriffslose und eine eingegriffene Konfiguration einzunehmen, wodurch erleichtert wird, die Vorrichtung (10; 100) an den Umfang eines Rings zu befestigen.

2. Vorrichtung nach Anspruch 1, wobei das flexible elastische oder verformbare Material des länglichen Bandes es ermöglicht, die Länge zwischen jeder Verankerung (30) um zwischen 10 und 40 %, vorzugsweise zwischen 20 und 30 % zu verlängern oder einzuziehen.

3. Vorrichtung nach Anspruch 1 oder 2, wobei das längliche Band (20) aus einem Material hergestellt ist, das ausgewählt ist aus: Geweben und Vliesen; elastischen Stoffen, wie etwa Dacron; Polyethylenterephthalat; Polyurethan; einem thermoplastischen Elastomer; gewebtem Velours; Polypropylen; Polytetrafluorethylen; heparinbeschichtetem Stoff; einem Silicon oder orientiertem Polyethylen; und biologischem Gewebe.

4. Vorrichtung nach einem der vorhergehenden Ansprüche, wobei die Verankerung (30) ein Metall- oder ein Polymermaterial umfasst.

5. Vorrichtung nach einem der vorhergehenden Ansprüche, wobei die Verankerung (30) eine Formgedächtnislegierung umfasst.

6. Vorrichtung nach Anspruch 5, wobei die Formgedächtnislegierung Nitinol umfasst.

7. Vorrichtung nach Anspruch 5 oder 6, wobei die Verankerung (30) in der Lage ist, durch Anwendung von Wärmeenergie von der eingriffslosen Konfiguration in die eingegriffene Konfiguration überzugehen.

8. Vorrichtung nach Anspruch 5 oder 6, wobei die Verankerung (30) in der Lage ist, durch ein Reduzieren von Wärmeenergie von der eingegriffenen Konfiguration in die eingriffslose Konfiguration überzugehen.

9. Vorrichtung nach Anspruch 4, wobei die Verankerung (30) durch Anwenden von mechanischer Kraft von der eingriffslosen Konfiguration in die eingegriffene Konfiguration oder von der eingegriffenen Konfiguration in die eingriffslose Konfiguration umgewandelt wird.

10. Vorrichtung nach Anspruch 9, wobei mechanische Kraft unter Verwendung eines Verankerungsumwandlungshilfsmittels auf die Verankerung (30) angewendet wird.

11. Vorrichtung nach einem der vorhergehenden Ansprüche, wobei die Verankerung (30) eine Klammer umfasst.

12. Vorrichtung nach Anspruch 9 oder 10, wobei die Verankerung einen/eine spiralförmigen/spiralförmige Draht oder Schraube umfasst.

13. Vorrichtung nach einem der vorhergehenden Ansprüche, wobei der Ring eine Klappe oder ein Sphinkter ist, vorzugsweise wobei die Klappe eine Herzklappe ist.

14. Vorrichtung nach einem der vorhergehenden Ansprüche, wobei die mehreren Verankerungen (30), wenn sie in der eingriffslosen Konfiguration sind, gleichmäßig entlang der Länge des länglichen Bandes (20) beabstandet sind.

15. Vorrichtung nach einem der vorhergehenden Ansprüche, wobei das längliche Band (20) mehrere vorgegebene Spaltpositionen (40) entlang seiner Länge umfasst, was es dem Band (20) ermöglicht, vor dem Gebrauch und/oder in Situ derart in der Länge verringert zu werden, dass das Positionieren der Vorrichtung (10; 100) im Herzgewebe erleichtert wird.

## Revendications

1. Dispositif d'annuloplastie (10 ; 100) pour une utilisation dans la réparation des tissus de la valvule cardiaque, comprenant :
une bande allongée (20) comprenant un matériau élastique souple ou déformable et ayant une pluralité d'ancrages (30) espacés à des intervalles prédéterminés sur la longueur de la bande (20),
dans lequel le matériau élastique souple ou déformable de la bande allongée permet à la longueur entre chaque ancrage (30) d'être allongée et rétractée entre 5 et 50 %, **caractérisé en ce que** chaque ancrage (30) est capable d'adopter une configuration non engagée et engagée, facilitant ainsi la fixation du dispositif (10 ; 100) à la périphérie d'un anneau.

2. Dispositif selon la revendication 1, dans lequel le matériau élastique souple ou déformable de la bande allongée permet à la longueur entre chaque ancrage (30) d'être allongée et rétractée entre 10 et 40 %, de préférence entre 20 et 30 %.

3. Dispositif selon la revendication 1 ou 2, dans lequel la bande allongée (20) est fabriquée à partir d'un matériau choisi parmi : le tissu tissé et non-tissé ; les tissus élastiques tels que le Dacron ; le polyéthylène téréphtalate ; le polyuréthane ; un élastomère thermoplastique ; le velours tissé ; le polypropylène ; le polytétrafluoroéthylène ; le tissu enduit d'héparine ; un polyéthylène siliconé ou orienté ; ou un tissu biologique.

4. Dispositif selon l'une quelconque des revendications précédentes, dans lequel l'ancrage (30) comprend un matériau métallique ou polymère.

5. Dispositif selon l'une quelconque des revendications précédentes, dans lequel l'ancrage (30) comprend un alliage à mémoire de forme.

6. Dispositif selon la revendication 5, dans lequel l'alliage à mémoire de forme comprend le Nitinol.

7. Dispositif selon la revendication 5 ou 6, dans lequel l'ancrage (30) est capable de passer de la configuration non engagée à la configuration engagée par application d'énergie thermique.

8. Dispositif selon la revendication 5 ou 6, dans lequel l'ancrage (30) est capable de passer de la configuration engagée à la configuration non engagée par réduction de l'énergie thermique.

9. Dispositif selon la revendication 4, dans lequel l'ancrage (30) passe de la configuration non engagée à la configuration engagée ou de la configuration engagée à la configuration non engagée par application de force mécanique.

10. Dispositif selon la revendication 9, dans lequel la force mécanique est appliquée sur l'ancrage (30) à l'aide d'un outil de conversion pour ancrage.

11. Dispositif selon l'une quelconque des revendications précédentes, dans lequel l'ancrage (30) comprend une agrafe.

12. Dispositif selon la revendication 9 ou 10, dans lequel l'ancrage comprend un fil ou une vis hélicoïdal(e).

13. Dispositif selon l'une quelconque des revendications précédentes, dans lequel l'anneau est une valvule ou un sphincter, de préférence dans lequel la valvule est une valvule cardiaque.

14. Dispositif selon l'une quelconque des revendications précédentes, dans lequel la pluralité d'ancrages (30) sont régulièrement espacés sur la longueur de la bande allongée (20) en cas de configuration non engagée.

15. Dispositif selon l'une quelconque des revendications précédentes, dans lequel la bande allongée (20) comprend une pluralité de positions de clivage prédéterminées (40) sur sa longueur, permettant à la bande (20) d'être réduite en longueur avant utilisation et/ou *in situ* afin de faciliter le positionnement du dispositif (10 ; 100) dans le tissu cardiaque.
